# EUROPÄISCHE PATENTSCHRIFT

(11) **EP 3 658 065 B1**
(45) Veröffentlichungstag und Bekanntmachung des Hinweises auf die Patenterteilung: **26.07.2023**
(21) Anmeldenummer: 18782935.3
(22) Anmeldetag: 01.10.2018
(51) Int. Cl.: A61C 5/62, A61C 5/66, A61C 3/00, A61K 6/00

(54) **MIKRO-APPLIKATOR ZUR VERWENDUNG MIT DENTALEN ADHÄSIVEN**
MICRO-APPLICATOR FOR USE WITH DENTAL ADHESIVES
MICRO-APPLICATEUR DESTINÉ À ÊTRE UTILISÉ AVEC DES ADHÉSIFS DENTAIRES

(30) Priorität: 04.10.2017 DE 102017122990
(43) Veröffentlichungstag der Anmeldung: 03.06.2020
(73) Patentinhaber: Kulzer GmbH, 63450 Hanau (DE)
(72) Erfinder: KONDZIELA, Mike, 35781 Weilburg (DE); KEMENY, Andrea, 61381 Friedrichsdorf (DE)
(74) Vertreter: Bendele, Tanja
(86) Internationale Anmeldenummer: PCT/EP2018/076603
(87) Internationale Veröffentlichungsnummer: WO 2019/068621

(56) Entgegenhaltungen:
- WO-A1-00/45732
- WO-A1-02/30314
- WO-A1-2012/154052
- US-A- 4 648 532
- US-A- 6 059 570
- US-A1- 2016 324 608

## Beschreibung

Die Erfindung betrifft einen dentalen Mikro-Applikator zur Applikation einer Flüssigkeit oder Paste in einer Kavität eines natürlichen Zahnes mit einem hohlen, hülsenförmigen Applikationskopf mit einem distalen und einem proximalen Bereich und mit einem Applikationsbereich an der Stirnseite am proximalen Bereich des Applikationskopfes, wobei in dem hohlen, hülsenförmigen Applikationskopf im proximalen Bereich mindestens ein Stanzdorn, dessen Spitze nach distal ausgerichtet ist, angeordnet ist, und an der Stirnseite am proximalen Bereich eine Öffnung für den Durchtritt einer zu applizierenden Flüssigkeit oder Paste ausgebildet ist, wobei in den hohlen, hülsenförmigen Applikationskopf von distal ein Mikrobehälter, insbesondere ein rohrförmiger Mikrobehälter eingebracht ist, insbesondere eingeschoben oder eingedreht ist, dessen Außenform mit Passung zur Innenform des Applikationskopfes ausgebildet ist, und wobei der Mikrobehälter, insbesondere der rohrförmige Mikrobehälter, einen Innenraum zur Aufnahme der zu applizierenden Flüssigkeit oder Paste aufweist. Ferner ist der Mikrobehälter an seiner distalen Seite und an seiner proximalen Stirnseite verschlossen.

Zur Verbesserung der Haftung dentaler Materialien an Zähnen, sind vielfältige Bondings- und Primer-Kombinationen bekannt. Die Haftmittel der jüngeren Generation umfassen einkomponentige, selbstätzende Haftmittel. Um eine gute Haftung auf dem Zahn zu gewährleisten, muss eine große, gut benetzbare Haftfläche bereitgestellt werden. Das Adhäsiv selbst muss dünnflüssig sein, gute benetzende Eigenschaften aufweisen und sehr schnell aushärten, um die Möglichkeit eines Kontaktes mit Feuchtigkeit, mit der Folge einer verminderten Haftung, zu minimieren.

So sind selbstätzende Adhäsive bekannt, die die Schritte der Säureätzung und des anschließenden Applizierens des Adhäsivs, in einem Schritt zusammenfassen. Das säurehaltige Adhäsivsystem löst die Schmierschicht auf und legt das darunterliegende Dentin frei oder löst die Schmierschicht lediglich an, um diese permeabel für Inhaltsstoffe des Adhäsivs zu machen. Simultan erfolgt eine Infiltration der Monomere in die Zahnhartsubstanz. Im Falle des Schmelzes wird durch die säurehaltigen Adhäsivsysteme ein der Phosphorsäureätzung ähnliches Ätzmuster erzeugt. Das zur Infiltration notwendige Lösungsmittel wird mit einem Luftpüster entfernt und das Adhäsiv wird gehärtet, bspw. strahlengehärtet. Analog werden Desensibilisierungsmittel auf dem Zahn oder in Zahnkavitäten angewendet.

Des Weiteren sind Einkomponenten-Applikatoren bekannt, die im Körper des Applikators ein Reservoir aufweisen, das durchgängig bis zum Applikationsmittel am proximalen Ende des Körpers reicht, wie in der WO02/30314A1. Alternative Applikatoren offenbart die EP1262151A1, die zusätzlich ein separates Auftragselement umfasst. WO2002/03014A1 offenbart eine Mehrkomponenten-Mischbehälter (10) zum Lagern und Ausbringen mit einem Gehäuse und einen verschiebbaren Hohlraumblock (20), der mindestens eine Kavität aufweist, die mit einem Spike korrespondiert. Der Hohlraumblock wird mittels eines Stempels verschoben.

Es besteht ein Bedarf an Mikro-Applikatoren, die mit einer Hand aktiviert werden können und direkt einsatzbereit sind. Zudem besteht die Aufgabe, einen Mikro-Applikator bereitzustellen, der direkt ein Auftragselement umfasst und so dimensioniert ist, dass der Applikator oder zumindest das Auftragselement in die zu behandelnde Kavität eines natürlichen Zahnes eines Patienten eingeführt werden kann. Daher bestand die Aufgabe der vorliegenden Erfindung darin, einen Mikro-Applikator zu entwickeln, der einhändig direkt in der Kavität eines natürlichen Zahnes verwendet werden kann. Ein besonderes Problem bei der Entwicklung eines solchen Mikro-Applikators, der in die Kavität eines Zahnes eingeführt werden können soll, liegt in den Anforderungen an die Dichtigkeit aller Komponenten trotz der notwendigen geringen Materialdicken sowie der geringen Dimensionierung der einzelnen Komponenten. Insoweit kommt der genauen Dimensionierung und insbesondere der Materialauswahl eine große Bedeutung zu.

Gelöst wird die Aufgabe mit einem dentalen Mikro-Applikator nach Anspruch 1 sowie einem Kit nach Anspruch 12.

Gegenstand der Erfindung ist ein dentaler Mikro-Applikator mit einem hohlen, hülsenförmigen Applikationskopf, der einen distalen und einem proximalen Bereich sowie einen Applikationsbereich an der Stirnseite des proximalen Bereiches des Applikationskopfes aufweist, wobei in dem hohlen, hülsenförmigen Applikationskopf im proximalen Bereich mindestens ein Stanzdorn, dessen Spitze nach distal ausgerichtet ist, angeordnet ist, insbesondere fixiert ist. Ferner ist in der Stirnseite des proximalen Bereiches des Applikationskopfes eine Öffnung mit einem Querschnitt (d₁) ausgebildet, für den Durchtritt einer zu applizierenden Flüssigkeit oder Paste, wobei in den hohlen, hülsenförmigen Applikationskopf von distal ein Mikrobehälter eingebracht ist, insbesondere ein rohrförmiger Mikrobehälter, eingeschoben oder eingedreht ist, dessen Außenform, insbesondere passend, zur Innenform des Applikationskopfes ausgebildet ist, insbesondere mit Übergangspassung oder Presspassung, und wobei der Mikrobehälter, insbesondere der rohrförmige Mikrobehälter, einen Innenraum, insbesondere mit einem Volumen von kleiner 1 mL, zur Aufnahme der zu applizierenden Flüssigkeit oder Paste aufweist, und, wobei der Mikrobehälter an seiner distalen Seite und an seiner proximalen Stirnseite verschlossen ist. Die Außenform des rohrförmigen Mikrobehälters mit Passung zur Innenform des Applikationskopfes ausgebildet. Der Querschnitt (d₁) der Öffnung des Applikationskopfes ist größer als der Querschnitt (d₄) im Mikrobehälter. Der Mikro-Applikator umfasst einen Stanzdorn mit einem Kanal. Der mindestens eine Stanzdorn kann mehrere Kanäle aufweisen, wobei der Querschnitt bzw. Gesamtquerschnitt der Kanäle vorzugsweise größer als der Querschnitt der Öffnung des Applikationskopfes ist. Der Mikro-Applikator kann im Applikationskopf im proximalen Bereich auch mehrere Stanzdorne aufweisen, die um die Öffnung in der proximalen Wand des Applikationskopfes angeordnet sind und deren Spitzen nach distal ausgerichtet sind. Der Mikro-Applikator kann in Alternativen 2 bis 20 Stanzdorne mit oder ohne Kanal umfassen.

Die Außenform des Mikrobehälters dem Positiv der als Negativ ausgebildeten Innenform des Applikationskopfes entspricht.

Erfindungsgemäß weist der Mikrobehälter, der von distal in den hohlen, hülsenförmigen Applikationskopf eingebracht ist, insbesondere eingeschoben oder eingedreht ist, eine Außenform mit Passung zur Innenform des Applikationskopfes auf.

Dabei kann der Mikrobehälter des Applikators zum Ausbringen der Flüssigkeit oder Paste, bei dem die Innenform des hohlen, hülsenförmigen Applikationskopfs in Passung zu der Außenform des Mikrobehälters, insbesondere des rohrförmigen Mikrobehälters, ausgebildet ist, so in den Applikationskopf eingeschoben oder eingedreht, vorzugsweise eingeschoben, werden, bis die proximale Stirnseite, insbesondere als Stirnfläche der Seitenwände des Mikrobehälters oder die Folie oder zerbrechliche Schicht des Mikrobehälters, insbesondere des rohrförmigen Mikrobehälters, innen an den Sockel des Stanzdorns oder an die proximale Innenwand des Applikationskopfes anstößt. Eine bevorzugte Ausführungsform des Eindrehens ist das Einschrauben. Beim Einschieben oder Eindrehen des Mikrobehälters in den Applikationskopf wird die Folie oder zerbrechliche Schicht an der proximalen Seite des Mikrobehälters, insbesondere des rohrförmigen Mikrobehälters, vom Stanzdorn zerstört, insbesondere durchstoßen oder abgetrennt. Dabei umfasst der Begriff zerstören sowohl ein Abtrennen der Folie oder der Schicht als auch ein Durchstoßen. Der Mikrobehälter und der innere Querschnitt der Seitenwand des Mikrobehälters sowie der Außenquerschnitt des Stanzdorns sind so ausgeführt, dass der Mikrobehälter außen über den Stanzdorn schiebbar ist, insbesondere weist der Stanzdorn einen Kanal auf, der vorzugsweise durch einen Sockel verläuft. Aufgrund der vorgenannten Passung wird gewährleistet, dass der Übergang zwischen Mikrobehälter und Applikationskopf abgedichtet ist und keine Flüssigkeit an dieser Stelle austreten kann. Die zu applizierende Flüssigkeit oder Paste tritt aus dem Innenraum nur durch die Öffnung im proximalen Bereich des Applikationskopfes aus. Der Mikrobehälter kann auch eine Mehrzahl an Stanzdornen aufweisen, so dass alle Ausführungen auch für diese Stanzdorne gelten. Zudem kann der jeweilige Stanzdorn einen oder mehrere Kanäle aufweisen, so dass die Ausführungen für den einen Kanal auch für diese Kanäle gelten.

Der Mikrobehälter ist vorzugsweise ein an der distalen Stirnseite und an der proximalen Stirnseite verschlossenes Hohlprofil. Bevorzugt ist a) ein rohrförmiger Mikrobehälter oder b) der Mikrobehälter hat ein Hohlprofil, das keinen kreisrunden Querschnitt aufweist, oder c) das Hohlprofil weist einen elliptischen, ovalen, dreieckigen oder rechteckigen Querschnitt sowie entsprechende dazwischenliegende Querschnitte auf. Der hohle, hülsenförmige Applikationskopf ist vorzugsweise in Passung zu der Außenform des Mikrobehälters, insbesondere des rohrförmigen Mikrobehälters oder des Mikrobehälters mit den genannten Hohlprofilen ausgebildet. Der Mikrobehälter kann ein Außengewinde oder ein Innengewinde und der Applikationskopf das entsprechende Gegengewinde ausgewählt aus Innen- und Außengewinde aufweisen. Alternativ können der Mikrobehälter und/oder der Applikationskopf mindestens eine Führungsnut, insbesondere eine I- oder L-förmige Führungsnut, sowie eine darin eingreifende Nase aufweisen.

Erfindungsgemäß ist die Dimensionierung des dentalen Mikro-Applikators im Applikationsbereich des Applikationskopfs im proximalen Bereich so angepasst, dass der Applikationsbereich und vorzugsweise der Applikationskopf in eine Kavität eines natürlichen Zahns eingeführt werden kann. Unter einer Kavität wird eine gebohrte Kavität im natürlichen Zahn verstanden. Erfindungsgemäß soll der Applikationsbereich und vorzugsweise der Applikationskopf des Mikro-Applikators am Kavitätenrand vorbei in die Kavität eingeführt werden können. Des Weiteren sollte der Mikro-Applikator optional zusätzlich in einer knick- und/oder biegbaren Ausführungsform bereitgestellt werden können. Die Hohlräume bzw. Kavitäten in der Zahnmedizin sind in Kavitätsklassen eingeteilt, wie I Grübchen und Fissuren in der Kaufläche (Seitenzähne okklusal), II Berührungsflächen der Backenzähne (Seitenzähne approximal und gegebenenfalls okklusal), III Berührungsflächen von Schneide- und Eckzähnen, Schneidekante nicht beteiligt, IV wie III, jedoch Schneidekante beteiligt, V Zahnhalsfläche und VI Defekte an den Milchzähnen.

Der äußere und optional der innere Durchmesser oder Querschnitt des Mikrobehälters, und insbesondere der Querschnitt der Innenform des Applikationskopfes, und der Querschnitt oder der Durchmesser der Öffnung sind dabei so bemessen, dass eine Flüssigkeit, insbesondere eine Flüssigkeit einer Zusammensetzung umfassend organische Lösemittel, und optional mindestens ein Monomer und optional Wasser, selbstständig aus dem Innenraum und damit aus dem Applikator bei annähernd senkrechter Haltung herausfließt und vorzugsweise die Vielzahl an Filamenten, die insbesondere einen Pinsel bilden, benetzt. Das Volumen des Innenraumes fasst vorzugsweise maximal 1 mL, insbesondere von 0,02 mL bis 0,9 mL, bevorzugt von 0,1 mL bis 0,9 mL.

Der Außendurchmesser (a) oder die längste Seite des Querschnitts (a) des Applikationskopfes beträgt von 1,0 mm bis 5,0 mm, insbesondere 1,0 mm bis 4,5 mm, insbesondere von 1,0 mm bis 4,0 mm, bevorzugt von 2,0 bis 4,0 mm, insbesondere jeweils vorstehend unabhängig mit +/- 0,1 mm, besonders bevorzugt um 3,5 mm +/- 0,5 mm.

Die Länge (b) der äußeren Seitenwand des Applikationskopfes beträgt vorzugsweise von 1 mm bis 75 mm, insbesondere von 2 mm bis 7,5 mm, bevorzugt von 3 mm bis 3,5 mm. Die Länge (c) der äußeren Seitenwand des Mikrobehälters beträgt vorzugsweise von 2 mm bis 75 mm, vorzugsweise 5 bis 40 mm, insbesondere wenn der Mikrobehälter auch die Funktion eines Griffs übernimmt, alternativ können auch von 5 mm bis 8 mm bevorzugt sein. Sofern der Mikrobehälter zusätzlich als Griff dient kann dieser an der distalen Seite massiv ohne Innenraum ausgebildet sein.

Die jeweilige Wandstärke der Seitenwand des Applikationskopfes und/oder des Mikrobehälters betragen vorzugsweise jeweils unabhängig von 0,1 mm bis 1 mm, insbesondere von 0,2 bis 0,6 mm, besonders bevorzugt von 0,3 bis 0,5 mm, vorzugsweise um 0,4 mm +/- 0,05 mm.

Der Innenraum des Mikrobehälters fasst ein Volumen von 0,04 mL bis kleiner gleich 1 mL, vorzugweise mit einem Volumen von kleiner gleich 0,75 mL, kleiner gleich 0,5 mL und dient vorzugsweise der Aufnahme einer mindestens ein organisches, insbesondere volatiles bzw. flüchtiges Lösemittel enthaltenden Flüssigkeit, vorzugsweise einer flüssigen dentalen Zusammensetzung.

Nach einer bevorzugten Alternative der Erfindung ist der Mikrobehälter, insbesondere der rohrförmige Mikrobehälter, um die Distanz (I₀), insbesondere um 6 mm, vorzugsweise von 2 mm bis 5 mm, innen in den hohlen Applikationskopf, insbesondere den hohlen rohrförmigen Applikationskopf, mit Passung, insbesondere in Übergangspassung oder Presspassung, eingebracht, insbesondere eingeschoben und/oder eingedreht. Des Weiteren ist der rohrförmige Mikrobehälter zusätzlich zur Distanz I₀ um die Distanz (I₁), insbesondere um mindestens 2 mm, vorzugsweise um mindestens 2 mm bis 5 mm, innen in den hohlen Applikationskopf, insbesondere den rohrförmigen Applikationskopf, mit Passung, insbesondere in Übergangspassung oder Presspassung, einbringbar, insbesondere einschiebbar oder eindrehbar. Der Mikrobehälter kann nach einer Ausführungsform eingedreht werden. Der Mikrobehälter kann vorzugsweise aus einem elastischen oder spröden Material gebildet sein. Nach einer weiteren Alternative kann ein rohrförmiger Mikrobehälter über eine Drehbewegung, insbesondere eine axiale Rotationsbewegung, bevorzugt eine Schraubbewegung, um die Distanz (I₁) zusätzlich zur Distanz I₀) innen in den hohlen Applikationskopf in Übergangspassung oder Presspassung eindrehbar sein, insbesondere einschraubbar sein.

Der Innenraum des rohrförmigen Mikrobehälters ist an seiner proximalen Stirnseite mit einer Folie oder einer zerbrechlichen Schicht verschlossen. Die Folie oder die zerbrechliche Schicht wird mit dem mindestens einen Stanzdorn durchstoßen, so dass die Flüssigkeit aus dem Innenraum des Mikrobehälters austreten kann. Die zerbrechliche Schicht kann auch durch Einwirkung einer äußeren Kraft auf den Applikationskopf zerstörbar sein.

Nach einer weiteren besonders bevorzugten Ausführungsform ist das Verhältnis Distanz I₁, um die der Mikrobehälter um die Distanz I₁ zusätzlich zur Distanz I₀ innen in den hohlen Applikationskopf in Übergangspassung oder Presspassung einschiebbar oder eindrehbar ist, zur Länge c der äußeren Seitenwand des Mikrobehälters 1 zu 2 bis 1 zu 75, vorzugsweise 1 zu 5 bis 1 zu 25.

Nach einer bevorzugten Ausführungsform weist die Länge (b) der äußeren Seitenwand des Applikationskopfes zur Länge (c) der äußeren Seitenwand des Mikrobehälters (b zu c) ein Verhältnis von 1 zu 1,2 bis 1 zu 25 auf, insbesondere von 1 zu 1,3 bis 1 zu 6, vorzugsweise von 1 zu 1,5 bis 1 zu 4. Des Weiteren weist vorzugsweise die gemittelte Länge (e) des jeweiligen Stanzdorns, optional mit Sockel, zur Länge (c) der äußeren Seitenwand des Mikrobehälters als Verhältnis von b zu c, ein Verhältnis von 1 zu 2 bis 1 zu 20 auf, vorzugsweise von 1 zu 2,5 bis 1 zu 10. Der erfindungsgemäße Stanzdorn ist explizit nicht als Verdrängerkolben ausgebildet, sondern dient alleinig der Zerstörung der Folie oder der zerbrechlichen Schicht.

Der Stanzdorn kann verschiedene Geometrien aufweisen; so kann der Stanzdorn ein einzelner Stift mit einer Spitze sein und neben der Öffnung in der Stirnseite angeordnet sein. Alternativ kann der Stanzdorn mit Spitze einen proximalen Sockel aufweisen, mit dem der Stanzdorn über eine Passung, insbesondere mit Übergangs- oder Presspassung, der Außenform des Sockels in der Innenform des hülsenförmigen Applikationskopfes im proximalen Bereich des Applikationskopfes fixiert ist. Vorzugsweise weist der Stanzdorn einen von distal nach proximal durchgängigen Kanal mit dem Querschnitt (d₄) auf, insbesondere beträgt der Querschnitt von 0,2 mm² bis 4 mm², bevorzugt von 0,4 mm² bis 1 mm². Besonders bevorzugt weist auch der Stanzdorn mit Sockel einen entsprechenden durchgängigen Kanal auf. Alternativ kann der jeweilige Stanzdorn mindestens einen von distal nach proximal durchgängigen Kanal, vorzugsweise zwei bis 20 Kanäle, mit dem Gesamtquerschnitt über alle Kanäle (d₄) aufweisen, insbesondere beträgt dieser Querschnitt von 0,2 mm² bis 4 mm², bevorzugt von 0,4 mm² bis 1 mm². Nach einer weiteren Alternative kann der Sockel zwei bis 20 Stanzdorne mit je einem Kanal aufweisen. In diesen beiden Alternativen weist auch der Sockel in den Alternativen a) Sockel mit Stanzdorn und zwei bis 20 Kanälen im Stanzdorn oder b) Sockel mit zwei bis 20 Stanzdornen mit je einem Kanal eine korrespondierende Anzahl von 2 bis 20 entsprechend durchgängiger Kanäle auf.

Nach einer weiteren alternativen Ausführungsform weist der Stanzdorn distal eine Spitze auf. In einer Alternative kann die Spitze gebildet werden durch eine gewölbte Ebene, vorzugsweise eine konkave oder planare Ebene, in welcher der Stanzdorn, insbesondere der Stanzdorn mit oder ohne Kanal, am distalen Ende ausgebildet ist. Vorzugsweise weist der Dorn an der Spitze eine Schneidkante auf. Entsprechendes gilt für den jeweiligen Stanzdorn, wenn es mehrere Stanzdorne gibt.

Ferner kann der Mikrobehälter an seiner distalen Seite gebogen, U-förmig und/oder als Griff ausgebildet sein. Vorzugsweise weist der Griff umlaufende rillenförmige Vertiefungen oder Noppen auf. Nach einer weiteren bevorzugten Ausführungsform kann die distale Seite des rohrförmigen Behälters verschweißt und/oder verpresst sein.

Im Applikationsbereich an der Stirnseite im proximalen Bereich des Applikationskopfes weist der Mikro-Applikator eine Vielzahl an Filamenten, insbesondere zur Ausbildung eines Pinsels auf. Die bevorzugte Mindestlänge (f) der Filamente beträgt von 2 mm bis 8 mm, insbesondere von 3 mm bis 7 mm, bevorzugt von 4 mm bis 6 mm. Vorzugsweise beträgt der Durchmesser der Filamente jeweils unabhängig voneinander im distalen Bereich von 0,2 mm bis 0,4 mm, insbesondere 0,3 mm +/- 0,25 mm, und optional im proximalen Bereich an der Spitze 0,05 mm bis 0,3 mm, insbesondere 0,2 mm mit +/- 0,1 mm. Dabei sind die Filamente vorzugsweise derart ausgestaltet, dass die Vielzahl der Filamente zur Ausbildung eines Pinsels eine Kapillarwirkung auf die Flüssigkeit oder Paste ausüben, so dass nach dem Zerstören der Folie oder der zerbrechlichen Schicht eine in dem Mikro-Behälter bevorratete Flüssigkeit in den Applikationsbereich überführt wird, wenn der Applikator, insbesondere der Applikationskopf, annähernd vertikal, insbesondere in einem Winkel von 70 bis 110°, vorzugsweise 80 bis 100° in Bezug zur Horizontalen ausgerichtet ist, um die Flüssigkeit mit dem Pinsel applizierbar zu machen.

In einer besonders bevorzugten Alternative ist die Innenform des hohlen, hülsenförmigen Applikationskopfs in Passung zu der Außenform des Mikrobehälters, insbesondere des rohrförmigen Mikrobehälters, entsprechend so ausgebildet, dass der Mikrobehälter in den Applikationskopf bis an die innere proximale Stirnseite eingeschoben werden kann, insbesondere bis zur Stirnfläche der Seitenwände, oder die Folie oder zerbrechliche Schicht des Mikrobehälters, insbesondere des rohrförmigen Mikrobehälters, innen an den Sockel des Stanzdorns oder an die proximale Innenwand des Applikationskopfes anstößt, wobei a) der Winkel alpha (α) zwischen der Mittelachse in der distalen Ebene am distalen Bereich des hohlen, hülsenförmigen Applikationskopfes gegenüber der Längsmittelachse im proximalen Bereich alpha kleiner gleich 180° beträgt, insbesondere beträgt der Winkle alpha von 175° bis 110°, insbesondere von 170° bis 130°, oder b) der Mikrobehälter, insbesondere der rohrförmige Mikrobehälter weist eine Soll-Knickstelle auf, insbesondere ist der Behälter oder die Seitenwand des Mikrobehälters an dieser Stelle dünner gegenüber dem restlichen Behälter oder der übrigen Seitenwand ausgeführt, oder der distale Bereich des Mikrobehälters, insbesondere des rohrförmigen Mikrobehälters, der nach dem Einschieben des Mikrobehälters in den Applikationsbereich distal übersteht, ist zusammendrückbar oder zusammendrückbar und biegbar. Vorzugsweise kann nach dem Zerstören der Folie oder der zerbrechlichen Schicht der distal überstehende Bereich des Mikrobehälters zusammendrückbar und/oder biegbar sein. Die proximale Innenwand, insbesondere Wand, des Applikationskopfes weist vorzugsweise die Öffnung an der Stirnseite des proximalen Bereiches auf. Die Öffnung in der Stirnseite des proximalen Bereiches kann vorzugsweise den gleichen Querschnitt wie der mindestens eine Kanal im Stanzdorn und optional im Sockel aufweisen.

Gegenstand der Erfindung ist ferner ein Applikator in dem i) die Folie oder die zerstörbare Schicht aus einem der folgenden Materialien ausgewählt ist umfassend Metalle, wie Aluminium, Legierungen, organischen Polymeren, wie PET (Polyethylentherephthalat), PE (Polyethylen), Polypropylen (PP), COC (Cycloolefin-Copolymer), COP (Cyclooelefin-Polymer), Acrylnitril-Butadien-Kautschuk, Ethylen-Vinylalkohol Polymer (EVOH, EVAL), halogenierte Polymere, Chloropren-Kautschuk, Perfluor-Kautschuk, HDPE, LDPE, poröses expandiertes Polypropylen (PEPP) und/oder expandiertes Polypropylen (EPP), PA (Polyamid), Glas, Hybridmaterial oder ein Verbundmaterial der vorgenannten Materialien, insbesondere ist die Folie oder die Schicht eine verschweißte oder kaschierte Aluminiumfolie, COC, COP, PP, EVOH oder PET-Folie, und/oder
ii) der Applikationskopf aus einem der folgenden Materialien ausgewählt ist, umfassend thermoplastische Elastomere, wie Copolyamide, Polyesterelastomere, PP/EPDM (EPDM: Ethylen-Propylen-Dien-Kautschuk), Styrol-Blockcopolymere, wie SBS, SEBS, SEPS, SEEPS und MBS, thermoplastische Elastomere auf Urethanbasis, Polyethylen, Polypropylen, Silikon, Polyamid (PA) oder Gummi, insbesondere sind der Applikationskopf und die Filamente aus dem gleichen Material, bevorzugt bestehen Applikationskopf und Filamente aus dem gleichem Material und sind als eine integrale Komponente ausgebildet, und/oder
iii) das Material aus dem der Mikrobehälter ausgewählt ist, umfasst Polyethylen, Polypropylen, Ethyl-Vinylalkohol, Cycloolefin-Polymeren (COP) und/oder Cycloolefin-Copolymeren (COC), besonders bevorzugt Copolymere von Ethan mit Cycloolefinen, wie Norbonen, Norbornen-Derivat, Tetracyclododecen, oder Polymeren von Cycloocten, halogenierten Polymeren, HDPE, LDPE, poröses expandiertes Polypropylen (PEPP) und/oder expandiertes Polypropylen (EPP) und/oder metallischen Folien oder ein Verbundmaterial der vorgenannten Materialien, und/oder
iv) das Material des Stanzdorns und optional des Sockels jeweils unabhängig aus einem der folgenden Materialien ausgewählt sind, umfassend Metall, Legierung, Glas, Keramik, Hybridwerkstoff oder einem Kunststoff mit einem Elastizitätsmodul von größer gleich 1500 MPA nach ISO 527 oder größer gleich 1300 MPa nach ISO 178.

Der Applikationskopf und/oder der Mikrobehälter können vorzugsweise aus einem inerten Polymer, insbesondere einem thermoplastischen Polymer hergestellt sein, umfassend Cyclo-Olefin-Polymeren (COP), PE, PP, HDPE, LDPE, poröses expandiertes Polypropylen (PEPP) und/oder expandiertes Polypropylen (EPP). Erfindungsgemäß kann der Applikationskopf und/oder der Mikrobehälter aus thermoplastischem Polymer zusätzlich eine Sperrschicht aus EVOH aufweisen. Der Applikationskopf und/oder der Mikrobehälter und/oder die Filamente können vorzugsweise aus Polyamid oder auch aus einem inerten Polymer, insbesondere einem thermoplastischen Polymer hergestellt sein, umfassend Cyclo-Olefin-Polymeren (COP), PE, PP, HDPE, LDPE, poröses expandiertes Polypropylen (PEPP) und/oder expandiertes Polypropylen (EPP).

Unter Gummi wird vorliegend ein Natur- oder Synthesekautschuk verstanden, wie u.a. ein Styrol-Butadien-Kautschuk, Butadien-Kautschuk (BR), Acrylnitril-Butadien-Kautschuk (NBR), Butylkautschuk (IIR), Ethylen-Propylen-Dien-Kautschuk (EPDM), Chloropren-Kautschuck, (CR) und Polyisopren-Kautschuk (IR).

In Alternativen kann es vorstellbar sein, dass zusätzlich zu den Filamenten im Applikationsbereich mindestens eine Bürste, Schwamm, Beflockung, Kamm, Knäuel, Pinsel, Spatel, Mischpad und/oder poröses Mittel angeordnet ist.

Das Material aus dem der Behälter und optional die Folie oder die zerstörbare Schicht besteht, kann in einer Alternative vorzugsweise für COP und/oder COC ein Biegemodul von 1800 bis 2200 MPa (ISO 178) aufweisen, insbesondere um 2200 MPa +/- 100 MPa, und/oder eine Biegefestigkeit von 80 MPa bis 100 MPa, vorzugsweise von 94 MPa +/- 5 MPa (ISO 178), und/oder ein Zugmodul von 2200 MPa bis 2600 MPa, insbesondere von 2400 +/-100 MPa (ISO 527), und/oder eine Zugfestigkeit von 40 MPa bis 70 MPa, insbesondere von 60 MPa +/- 5 MPa (ISO 527), und/oder eine Zugdehnung von 10 MPa bis 30 MPa, insbesondere von 20 MPa (ISO 527).

Ferner ist Gegenstand der Erfindung ein Kit umfassend einen Mikro-Applikator, wobei in dem Mikrobehälter, insbesondere dem rohrförmigen Mikrobehälter, eine Flüssigkeit enthalten ist, insbesondere eine mindestens ein organisches Lösemittel enthaltende Flüssigkeit, vorzugsweise eine flüssige dentale Zusammensetzung enthaltend mindestens ein flüchtiges organisches Lösemittel und optional mindestens ein Monomer sowie optional Wasser und/oder optional eine organische Säure.

Gleichfalls ist Gegenstand der Erfindung ein Kit umfassend eine Umverpackung sowie eine Vielzahl an Mikro-Applikatoren. Bevorzugt ist die flüssige dentale Zusammensetzung ein Adhäsiv, ein lichthärtendes Adhäsiv, ein Ätzmittel, ein dentales Haftmittel.

Ebenso ist Gegenstand der Erfindung ein Applikator in dem der Innenraum mit einer dentalen Flüssigkeit gefüllt ist, insbesondere mit einem dentalen Adhäsiv, vorzugsweise mit einem selbstätzenden dentalen Adhäsiv, wie iBOND^{®} Self Etch. Ebenso Gegenstand der Erfindung ist die Verwendung eines erfindungsgemäßen Applikators zur Applikation von flüssigen, dentalen Zusammensetzungen, wie insbesondere iBOND^{®} Self Etch auf Zähnen. Ebenso Gegenstand der Erfindung ist ein Kit enthaltend ein selbstätzendes dentales Adhäsiv zur Verwendung bei der Behandlung einer Demineralisierung eines Zahnes bzw. Zähnen und/oder bei der Behandlung von Karies, vorzugsweise der Initialkaries und/oder Caries profunda.

Nach einer Ausführungsvariante ist Gegenstand der Erfindung ein Kit umfassend mindestens einen Applikator, wobei jeder Applikator einzeln in einer lichtundurchlässigen Verpackung bevorratet ist.

Erfindungsgemäße im Innenraum des Applikators bevorratete Adhäsive sind vorzugsweise selbstätzende dentale Adhäsive.

Bevorzugt umfasst die dentale Zusammensetzung, insbesondere das dentale Adhäsiv ein A) radikalisch polymerisierbares Monomer mit einer Säuregruppe im Molekül, B1) optional einen Photosensibilisator, und/oder optional B2) ein Peroxid, C) ein mit Wasser mischbares Lösemittel, wie Alkohol, Keton, Ester, Ketal, Isopropylidenglycerin, Ethanol, vorzugsweise Aceton; und D) Wasser.

Weiter kann das Kit umfassen E) mindestens ein radikalisch polymerisierbares Monomer ohne Säuregruppe, das vorzugsweise in Wasser nicht oder kaum löslich ist (kleiner 2 g/ 100 ml H₂O).

Die radikalisch polymerisierbaren Monomere mit einer Säuregruppe im Molekül gemäß A), auch saure Komponente genannt, umfassen polymerisierbare Monomere mit mindestens einer, vorzugsweise mehreren Ethylen-Gruppen sowie mindestens eine Carbonsäure-, Carbonsäureanhydridgruppe, Phosphorsäuregruppe und/oder Sulfonsäuregruppe.

Monofunktionelle polymerisierbare Monomere mit einer Carbonsäure- oder Carbonsäureanhydridgruppe im Molekül können ausgewählt sein aus Monocarbonsäuren, Dicarbonsäuren, Tricarbonsäuren, Tetracarbonsäuren, Polycarbonsäuren und Anhydriden davon. Bevorzugte Verbindungen können Carbonsäuren und/oder Anhydride sein wie Maleinsäure, p-Vinylbenzoesäure, 11-(Meth)acryloyloxy-1,1-undecandicarbonsäure (MAC-10), 1,4-Di(meth)acryloyloxyethylpyromellitsäure, 6-(meth)acryloyloxyethylnaphthalen-1,2,6-tricarbonsäure, 4-(Meth)acryloyloxymethyltrimellitsäure und Anhydride davon, 4-(Meth)acryloyloxyethyltrimellitsäure und dessen Anhydrid, 4-(Meth)acryloyloxybutyltrimellitsäure und dessen Anhydrid, 4-[2-Hydroxy-3-(meth)acryloyloxy]butyltrimellitsäure und dessen Anhydrid, 2,3-Bis(3,4-dicarboxybenzoyloxy)propyl(meth)acrylat, 2-, 3-, oder 4-(Meth)-acryloyloxybenzoesäure, N-O-Di(meth)acryloyloxytyrosin, O-(Meth)acryloyloxytyrosin, N-(Meth)acryloyloxytyrosin, N-(Meth)acryloyloxyphenylalanin, N-(Meth)acryloyl-p-amin-benzoesäure, N-(Meth)acryloyl-O-aminbenzoesäure, Addukt aus Glycidyl(meth)acrylat mit N-Phenylglycin oder N-tolylglycin, 4-[(2-Hydroxy-3-(meth)acryloyloxypropyl)amino]phthalsäure, 3- oder 4-[N-Methyl-N-(2-hydroxy-3-(meth)acryloyloxypropyl)amino]phthalsäure, (Meth)acryloylaminosalicylsäure und (Meth)acryloyloxysalicylsäure. Bevorzugt sind 11-(Meth)acryloyloxy-1,1-undecandicarbonsäure (MAC-10) und 4-Methacryloyloxyethyltrimellit-säure (4-MET) oder deren Anhydrid (4-META). Polyfunktionelle polymerisierbare Monomere mit mindestens zwei Carboxylgruppen im Molekül, die als Komponente A) verwendbar sind, können umfassen Dicarbonsäuren, Tricarbonsäuren, Tetracarbonsäuren und Derivate davon, wie beispielsweise ein Additionsprodukt aus 2-Hydroxyethyl(meth)acrylat und Pyromellitdianhydrid (PMDM), ein Additionsreaktionsprodukt aus 2 Mol Hydroxyethyl-(meth)acrylat und 1 Mol Maleinsäureanhydrid oder 3,3',4,4'-Benzophenontetracarbonsäuredianhydrid (BTDA) oder 3,3',4,4'-Biphenyltetracarbonsäuredianhydrid, und 2-(3,4-Dicarboxybenzoyloxy)1,3-di(meth)acryloyloxypropan.

Polymerisierbare Monomere mit mindestens einer Phosphorsäuregruppe im Molekül umfassen beispielsweise 2-(Meth)acryloyloxyethylsäurephosphat, 2- und 3-(Meth)acryloyloxypropylsäurephosphat, 4-(Meth)acryloyloxybutylsäurephosphat, 6-(Meth)-acryloyloxyhexylsäurephosphat, 8-(Meth)acryloyloxyoctylsäurephosphat, 10-(Meth)-acryloyloxydecylsäurephosphat, 12-(Meth)acryloyloxydodecylsäurephosphat, Bis(2-(meth)-acryloyloxyethyl)säurephosphat, Bis({2 oder 3}-(meth)acryloyloxypropyl)säurephosphat, 2-(Meth)acryloyloxyethylphenylsäurephosphat, 2-(Meth)acryloyloxyethyl-p-methoxyphenyl-säurephosphat und dergleichen. Die Phosphorsäure in diesen Verbindungen kann durch eine Thiophosphorsäuregruppe ersetzt sein.

Von den vorgenannten Monomeren sind die folgenden bevorzugt 2-(Meth)acryloyloxyethyl-phenylsäurephosphat und 10-(Meth)acryloyloxydecylsäurephosphat. Die Monomere mit einer Phosphorsäuregruppe können einzeln oder in einer Kombination verwendet werden.

Polymerisierbare Monomere mit einer Sulfonsäuregruppe im Molekül können umfassen 2-Sulfoethyl(meth)acrylat, 2- oder 1-Sulfo-1- oder -2-Propyl(meth)acrylat, 1- oder 3-Sulfo-2-butyl-(meth)acrylat, 3-Brom-2-sulfo-2-propyl(meth)acrylat, 3-Methoxy-1-sulfo-2-propyl(meth)-acrylat, 1,1-Dimethyl-2-sulfoethyl(meth)acrylamid und 2-Methyl-2-(meth)acrylamidpropan-sulfonsäure, bevorzugt ist 2-Methyl-2-(meth)acrylamidpropsansulfonsäure.

Das polymerisierbare Monomer A) kann eine Säure-Gruppe umfassen die als Salz, wie beispielsweise als monovalentes oder polyvalentes Metallsalz oder Ammoniumsalz vorliegt. Dabei ist es jedoch bevorzugt, wenn das Monomer A) als Säure wirkt, wenn sie in Verbindung mit einer anderen sauren Verbindung verwendet und mit der anderen sauren Verbindung kontaktiert wird. Die obigen Komponenten A) können einzeln oder in Kombination miteinander verwendet werden.

Bevorzugte Komponenten B1) und/oder B2) umfassen als B1) optional einen Photosensibilisator, und/oder optional B2) ein Peroxid.

Bevorzugte Komponenten (B1) umfassen eine alpha-Ketocarbonylverbindung oder Acylphosphinoxidverbindung. Konkret bevorzugt sind alpha-Diketon, alpha-Ketoaldehyd, alpha-Ketocarboxylsäure, alpha-Ketocarboxylat. Konkret bevorzugt sind alpha-Diketone, wie beispielsweise Diacetyl, 2,3-Pentadion, 2,3-Hexadion, Benzyl, 4,4'-Dimethoxybenzyl, 4,4'-Diethoxybenzyl, 4,4'-Oxybenzyl, 4,4'-Dichlorbenzyl, 4-Nitrobenzyl, alpha-Naphthyl, Campherchinon, Campherchinonsulfonsäure, Campherchinoncarbonsäure und 1,2-Cyclohexandion, alpha-Ketoaldehyde, wie beispielsweise Methylglyoxal und Phenylglyoxal, und andere, wie beispielsweise Pyruvinsäure, Benzoylameisensäure, Phenylpyruvinsäure, Methylpyruvat, Ethylbenzoylformiat, Methylphenylpyruvat und Butylphenylpyruvat.

Besonders bevorzugt sind alpha-Diketone aufgrund ihrer Stabilität, sowie Diacetyl, Benzyl und Campherchinon.

Des Weiteren umfasst die Komponente B1) Benzoyldimethoxyphosphinoxid, Benzoylethoxyphenylphosphinoxid, 2-Methylbenzoyldiphenylphosphinoxid, 2,4,6-Trimethylbenzoyldiphenylphosphinoxid sowie deren Derivate. Alle photoaktiven Verbindungen können einzeln oder im Gemisch verwendet werden.

Als Peroxid B2) können eingesetzt werden Verbindungen wie Diacetyldiperoxid, Dipropylperoxid, Dibutylperoxid, Dicaprylperoxid, Dilaurylperoxid, Benzoylperoxid (BPO), p,p'-Dichlorbenzoylperoxid, p,p'-Dimethoxybenzoylperoxid, p,p'-Dimethylbenzoylperoxid und p,p'-Dinitrodibenzoylperoxid; und anorganische Peroxide wie beispielsweise Ammoniumpersulfat, Kaliumpersulfat, Kaliumchlorat, Kaliumbromat und Kaliumperphosphat. Bevorzugt ist BPO.

Als Komponente C) ein mit Wasser mischbares Lösemittel können vorzugsweise verwendet werden Alkohol, Keton, Ester, Ketal, Isopropylidenglycerin, wie Ethanol, aber vorzugsweise Aceton.

Die erfindungsgemäße Komponente C) ist ein wasserlösliches organisches Lösungsmittel. Dieses dient zur gleichförmigen Auflösung oder Dispergierung jeder der obigen Komponenten;, das Lösemittel soll inert gegenüber den Komponenten und vorteilhaft flüchtig sein. Es können auch höhere Alkohole verwendet werden wie beispielsweise Ethylenglykol, Propylenglykol und Glycerin.

Die Komponente E) umfasst vorzugsweise mindestens ein radikalisch polymerisierbares Monomer ohne Säuregruppe, das insbesondere in Wasser nicht oder kaum löslich ist. Bevorzugte radikalisch polymerisierbare Monomere ohne Säuregruppe, die von der Komponente (A) verschieden sind, umfassen aromatische Vinylverbindungen, wie beispielsweise Styrol und Divinylbenzol, Vinylester wie beispielsweise Vinylacetat, aliphatische Ester von (Meth)acrylsäure, wie beispielsweise Methyl(meth)acrylat, Ethyl(meth)acrylat, Propyl(meth)acrylat, Butyl(meth)acrylat, Pentyl(meth)acrylat, Hexyl(meth)acrylat, Neopentylglykoldi(meth)acrylat und Trimethylolpropantri(meth)acrylat, aromatische Ester wie beispielsweise Phenyl(meth)acrylat, aromatische (Meth)acrylate wie beispielsweise 2-Hydroxy-3-phenoxypropyl(meth)acrylat, ein Addukt aus 1 Mol Bisphenol-A mit 2 Mol Glycidyl(meth)acrylat (Bis-GMA), ein Kondensat aus 1 Mol eines Additionspolymers aus Bisphenol-A mit Glycidylether und 2 Mol (Meth)acrylsäure und ein Kondensat aus 1 Mol eines Addukts aus Bisphenol-A mit Ethylenoxid und 2 Mol (Meth)acrylsäure (Anzahl der Additionsketten von Ethylenoxid m + n = 2,6), urethanbindungshaltige Methacrylate, wie beispielsweise 2-(Meth)acryloyloxyethylisocyanat und ein Addukt (UDMA) aus 2 Mol Hydroxyethyl(meth)acrylat mit 1 Mol 2,2,4-(oder 2,4,4-) trimethyl-1,6-hexamethylendiisocyanat; aliphatische (Meth)acrylsäureester wie beispielsweise 1,6-Hexamethylendimethacrylat (1,6-HX), Neopentylglykoldi(meth)acrylat und Trimethylolpropantri(meth)acrylat; Polyethylenglykoldi(meth)acrylate (Kettenlänge n = weniger als 6), wie beispielsweise Ethylenglykoldi(meth)acrylat, Diethylenglykol-di(meth)acrylat und Triethylenglykoldi(meth)acrylat, und Polypropylenglykoldi(meth)acrylate (Kettenzahl n = 12 oder weniger) wie beispielsweise Propylenglyko-di(meth)acrylat, Dipropylenglykoldi(meth)acrylat, Tripropylenglykoldi (meth)acrylat und Nanopropylenglykoldi(meth)acrylat. Erfindungsgemäße Komponenten E) umfassen Monomere, die mindestens eine (Meth)acrylat-Gruppe umfassen ausgewählt aus Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylenglykolmonomethacrylat, Tetrahydrofurylmethacrylat, Methylacrylat, Ethylacrylat, Propylacrylat, Butylacrylat, n-Hexylacrylat, 2-Phenoxyethylacrylat, Isobornylacrylat, Isodecylacrylat, Polypropylenglykolmonoacrylat, Tetrahydrofurylacrylat, Hydroxyethylacrylat, Hydroxy-propylacrylat, Hydroxyethylmethacrylat, Hydroxypropylmethacrylat, oder Mischungen davon oder mindestens einen Vernetzer umfassen. Typische Vernetzer sind BDMA, 1,4-Butandiol-dimethacrylat (1,4-BDMA) oder Pentaerythritoltetraacrylat, Urethandimethacrylat (UDMA), Bis-GMA-Monomer (Bisphenyl-A-Glycidylmethacrylat). Die Verwendung von Verdünnungsmitteln (dünnflüssige Acrylate wie Triethylenglykoldimethacrylat (TEGDMA) und Diethylenglykoldimethacrylat (DEGMA)), usw.

Bevorzugte (Meth)acrylat mit mindestens zwei (Meth)acrylat-Gruppen sind ausgewählt aus Ethandioldimethacrylat, Tetraethylenglykoldimethacrylat, Diethylenglykoldimethacrylat, Ethylenglykoldimethacrylat, Polyethylenglykoldimethacrylat (400) oder (600), Butandioldimethacrylat, Hexandioldimethacrylat, Decandioldimethacrylat, Dodecandioldimethacrylat, 1,3-Butylenglykoldimethacrylat, Dipropylenglykolmethacrylat, Bisphenol-A-dimethacrylat, Bisphenol-A-dimethacrylat-Derivate, wie ethoxyliertes 2-Bisphenol-A-dimethacrylat, Trimethylolpropantrimethacrylat, Triethylenglykoldimethacrylat, 2,2-Bis-4-(3-methacryloxy-2-hydroxypropoxy)phenylpropan (Bis-GMA), Tricyclodecandimethanoldimethacrylat, ein Urethanmethacrylat mit mindestens zwei Methacrylat-Gruppen oder einer Mischung enthaltend mindestens eines der (Meth)acrylate.

Bevorzugte (Meth)acrylat mit drei bis sechs (Meth)acrylat-Gruppen sind ausgewählt aus (i) mit drei (Meth)acrylat-Gruppen aus ethoxyliertem 15-Trimethylolpropantriacrylat, ethoxyliertem 5-Pentaerythritoltriacrylat, propoxyliertem 5.5-Glyceryltriacrylat, Trimethylolpropantrimethacrylat, Tris(2-hydroxyethyl)isocyanurattriacrylat, und/oder (ii) mit vier (Meth)acrylat-Gruppen aus Ditrimethylolpropantetraacrylat, ethoxyliertem4-Pentaerythritoltetraacrylat, Pentaerythritoltetraacrylat, Ditrimethylolpropantetramethacrylat, ethoxyliertem-4-Pentaerythritoltetramethacrylat, Pentaerythritoltetramethacrylat, und/oder (iii) mit fünf (Meth)acrylat-Gruppen aus Dipentaerythritol-pentaacrylat, i-Pentaerythritolpentamethacrylat, Dipentaerythritolpentaacrylat, Di(tetramethylolmethan)pentamethacrylat, und/oder (iv) mit sechs (Meth)acrylat-Gruppen aus einem Dipentaerythritolhexa(meth)acrylat. Ebenfalls geeignet sind Oligomere von (Meth)acrylaten, insbesondere Urethandiacrylat-Oligomer.

Nachfolgend wird die Erfindung anhand von Beispielen näher erläutert, ohne sie auf die Beispiele zu beschränken:

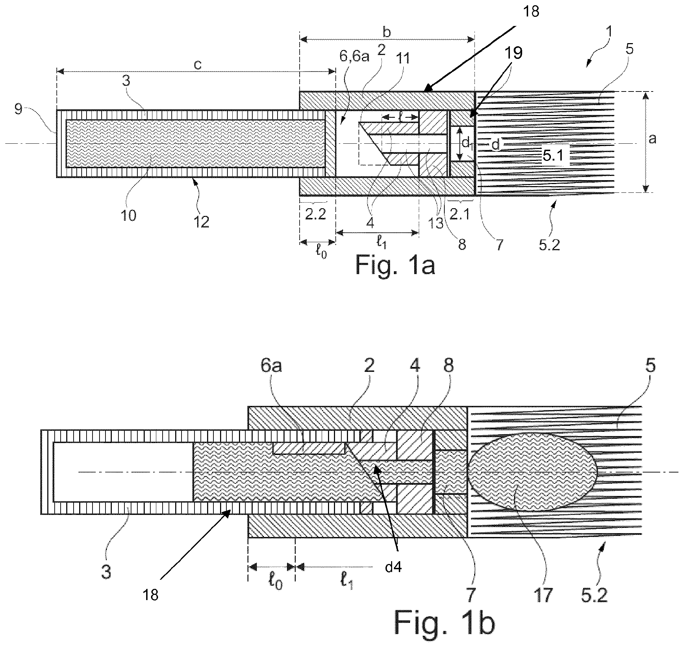

**Figuren 1a** und **1b** stellen dar einen dentalen Mikro-Applikator **1** mit einem hohlen, hülsenförmigen Applikationskopf **2,** der einen distalen **2.2** und einem proximalen Bereich **2.1** sowie einen Applikationsbereich **5.1** an der Stirnseite am proximalen Bereich des Applikationskopfes **2** aufweist. In dem Applikationskopf **2** ist im proximalen Bereich **2.1** ein Stanzdorn **4** angeordnet, dessen Spitze **11** nach distal ausgerichtet ist. Der Stanzdorn kann, in über eine Passung fixiert sein oder integral mit dem Applikationskopf ausgeformt sein. In der Stirnseite ist eine Öffnung **7** mit dem Durchmesser **d1** für den Durchtritt einer dentalen, flüssigen Zusammensetzung ausgebildet. Der Stanzdorn **4** weist einen Kanal mit einem Durchmesser **d4** auf. In den Applikationskopf **2** ist von distal der Mikrobehälter **3** eingeschoben und dichtet beide Komponenten gegeneinander ab. Im Innenraum **10** ist die dentale Zusammensetzung an der proximalen Stirnseite **6** über eine Folie **6a** gegenüber der Atmosphäre dicht verschlossen. Der Mikrobehälter **3** ist um die Distanz **I₀** innen in den hohlen rohrförmigen Applikationskopf **2** in Übergangspassung oder Presspassung eingeschoben und kann zusätzlich um die Distanz **I₁** innen in den hohlen, rohrförmigen Applikationskopf **2** in Übergangspassung oder Presspassung eingeschoben werden. Der Stanzdorn **4** mit Spitze **11,** und vorzugsweise Schneidkante, weist einen proximalen Sockel **8** auf. Der dargestellte Stanzdorn **4** weist einen von distal nach proximal durchgängigen Kanal **13** mit dem Querschnitt **d₄** auf. In **Figur 1b** ist die Folie **6a** geöffnet und die Flüssigkeit kann durch die Öffnung **7** in die Filamente als Tropfen **17** austreten. Der Mikro-Applikator kann alternativ einen Stanzdorn mit mehreren Kanälen oder mehrere Stanzdorne mit je mindestens einem Kanal aufweisen.

Der Mikrobehälter ist an seiner distalen Seite **9** als Griff ausgebildet. Des Weiteren zeigt die **Figur 1a** die Länge **a** des Querschnitts des Applikationskopfes, die Länge **b** der äußeren Seitenwand **18** des Applikationskopfes **2** sowie die Länge c der äußeren Seitenwand **12** des Mikrobehälters **3** als auch die gemittelte Länge **e** des Stanzdorns.

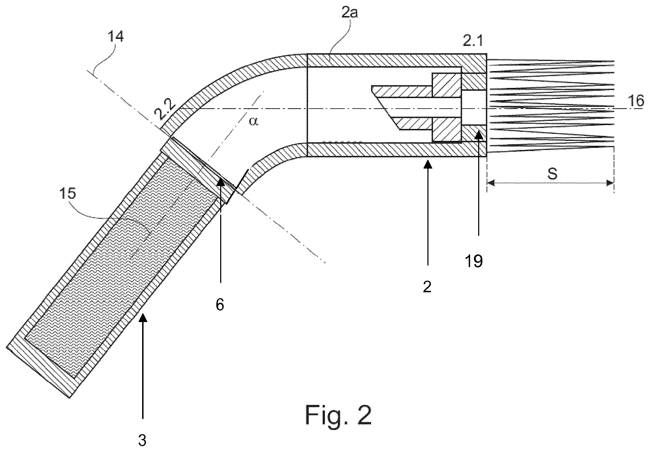

**Figur 2** zeigt einen Mikro-Applikator bei dem die Innenform des hohlen, hülsenförmigen Applikationskopfs **2** in Passung zu der Außenform des rohrförmigen Mikrobehälters **3** ausgebildet ist, so dass der Mikrobehälter **3** in den Applikationskopf **2** eingeschoben werden kann bis die proximalen Stirnseite **6** des rohrförmigen Behälters **3** innen an den Sockel **8** des Stanzdorns oder an die proximale Innenwand **19** des Applikationskopfes **2** anstößt, wobei a) der Winkel alpha (α) zwischen der Mittelachse **15** in der distalen Ebene **14** am distalen Bereich **2.2** des hohlen, hülsenförmigen Applikationskopfes **2** gegenüber der Längsmittelachse **16** im proximalen Bereich **2.1** alpha kleiner gleich 180° beträgt, insbesondere beträgt der Winkel kleiner 180°, vorzugsweise beträgt der Winkel alpha von 175° bis 110°.

### Bezugszeichen

- 1: Mikro-Applikator
- **2**: Applikationskopf, insbesondere hohler, hülsenförmiger Applikationskopf
- **2.1**: proximaler Bereich
- **2.2.**: distaler Bereich
- **3**: Mikrobehälter, insbesondere rohrförmiger Mikrobehälter
- **4**: Stanzdorn
- **5**: Filament
- **5.1**: Applikationsbereich
- **5.2.**: Pinsel
- **6**: proximale Stirnseite
- **6a**: Folie
- **6b**: Schicht, insbesondere zerbrechliche Schicht
- **7**: Öffnung
- **8**: Sockel
- **9**: distale Seite
- **10**: Innenraum
- **11**: Spitze
- **12**: Seitenwand des Mikrobehälters
- **13**: Kanal
- **14**: distale Ebene
- **15**: Mittelachse
- **16**: Längsmittelachse
- **17**: Tropfen der Flüssigkeit
- **18**: Seitenwand des Applikationskopfes
- **19**: proximale Innenwand, insbesondere Wand, des Applikationskopfes
- **a**: Länge des Querschnitts des Applikationskopfes
- **b**: Länge der äußeren Seitenwand **17** des Applikationskopfes **2**
- **c**: Länge der äußeren Seitenwand **12** des Mikrobehälters **3**
- **e**: gemittelte Länge des Stanzdorns
- **d1**: Querschnitt der Öffnung **7**
- **d4**: Querschnitt **d₄** des Kanals im Stanzdorn

ℓ₀ bzw. **I₀** Distanz innen, um die der Mikrobehälter in den hohlen rohrförmigen Applikationskopf **2** in Übergangspassung oder Presspassung im Lagerzustand eingebracht ist, insbesondere eingeschoben oder eingedreht ist
ℓ₁ bzw. **I₁** Distanz, um die der Mikrobehälter innen in den hohlen, rohrförmigen Applikationskopf **2** in Übergangspassung oder Presspassung zusätzlich zur Distanz I₀ eingebracht ist, insbesondere einschiebbar oder eindrehbar ist

## Patentansprüche

1. Dentaler Mikro-Applikator (1) mit einem hohlen, hülsenförmigen Applikationskopf (2) mit einem distalen (2.2) und einem proximalen Bereich (2.1) und mit einem Applikationsbereich (5.1) an der Stirnseite des proximalen Bereiches (2.1) des Applikationskopfes (2), wobei in dem hohlen, hülsenförmigen Applikationskopf (2) im proximalen Bereich (2.1) mindestens ein Stanzdorn (4), dessen Spitze (11) nach distal ausgerichtet ist, angeordnet ist, und in der Stirnseite des proximalen Bereiches (2.1) eine Öffnung (7) mit einem Querschnitt (d₁) für den Durchtritt einer zu applizierenden Flüssigkeit oder Paste ausgebildet ist, wobei in den hohlen, hülsenförmigen Applikationskopf (2) von distal ein Mikrobehälter (3) eingebracht ist, insbesondere eingeschoben oder eingedreht ist, dessen Außenform dem Positiv der als Negativ ausgebildeten Innenform des Applikationskopfes (2) entspricht, und wobei der Mikrobehälter (3) einen Innenraum (10) zur Aufnahme der zu applizierenden Flüssigkeit oder Paste aufweist, und, wobei der Mikrobehälter (3) an seiner distalen Seite (9) und an seiner proximalen Stirnseite (6) verschlossen ist, wobei die Länge (b) der äußeren Seitenwand (18) des Applikationskopfes (2) zur Länge (c) der äußeren Seitenwand (12) des Mikrobehälters (3) ein Verhältnis von 1 zu 1,2 bis 1 zu 25 aufweist, wobei der Stanzdorn (4) mit Spitze (11) einen proximalen Sockel (8) aufweist, mit dem der Stanzdorn (4) über eine Passung mit Übergangs- oder Presspassung der Außenform des Sockels (8) in der Innenform des hülsenförmigen Applikationskopfes (2) im proximalen Bereich des Applikationskopfes (2) fixiert ist und einen von distal nach proximal durchgängigen Kanal mit Querschnitt (d₄) aufweist, wobei der Querschnitt (d₁) der Öffnung des Applikationskopfes (2) größer ist als der Querschnitt (d₄).

2. Applikator nach Anspruch 1, **dadurch gekennzeichnet, dass** der Mikrobehälter (3) um die Distanz (I₀) innen in den hohlen Applikationskopf (2) in Übergangspassung oder Presspassung eingebracht ist, insbesondere eingeschoben oder eingedreht ist.

3. Applikator nach Anspruch 2, **dadurch gekennzeichnet, dass** a) der Mikrobehälter (3) um die Distanz (I₁) zusätzlich zur Distanz I₀ innen in den hohlen Applikationskopf (2) in Übergangspassung oder Presspassung einschiebbar ist oder b) ein rohrförmiger Mikrobehälter über eine Drehbewegung, um die Distanz (I₁) zusätzlich zur Distanz I₀ innen in den hohlen Applikationskopf (2) in Übergangspassung oder Presspassung eindrehbar ist.

4. Applikator nach einem der Ansprüche 1 bis 3, **dadurch gekennzeichnet, dass** der Mikrobehälter (3) an seiner proximalen Stirnseite (6) mit einer Folie (6a) oder einer zerbrechlichen Schicht (6b) verschlossen ist.

5. Applikator nach einem der Ansprüche 1 bis 4, **dadurch gekennzeichnet, dass** die Innenform des hohlen, hülsenförmigen Applikationskopfs (2) in Passung zu der Außenform des Mikrobehälters (3) ausgebildet ist, so dass der Mikrobehälter (3) in den Applikationskopf (2) einschiebbar oder eindrehbar ist, bis die proximale Stirnseite (6) des Mikrobehälters (3) innen an den Sockel (8) des mindestens einen Stanzdorns (4) oder an die proximale Innenwand (19) des Applikationskopfes (2) anstößt.

6. Applikator nach Anspruch 4 oder 5, **dadurch gekennzeichnet, dass** die Folie (6a), oder zerbrechliche Schicht (6b) an der proximalen Seite des Mikrobehälters (3), insbesondere eines rohrförmigen Mikrobehälters, beim Einschieben oder Eindrehen des Mikrobehälters in den Applikationskopf (2) vom mindestens einen Stanzdorn (4) zerstört oder abgetrennt wird.

7. Applikator nach einem der Ansprüche 1 bis 6, **dadurch gekennzeichnet, dass** im Applikationsbereich (5.1) an der Stirnseite im proximalen Bereich des Applikationskopfes (2) eine Vielzahl an Filamenten (5) zur Ausbildung eines Pinsels (5.2), insbesondere mit einer Mindestlänge (f) von 2 mm bis 8 mm, insbesondere 3 mm bis 7 mm, bevorzugt von 4 mm bis 6 mm ausgebildet sind.

8. Applikator nach Anspruch 7, **dadurch gekennzeichnet, dass** der Mikrobehälter ein an der proximalen und distalen Stirnseite verschlossenes Hohlprofil ist.

9. Applikator nach einem der Ansprüche 1 bis 8, **dadurch gekennzeichnet, dass**
a) der Mikrobehälter von distal in den hohlen, hülsenförmigen Applikationskopf (2) eingebracht ist, wobei die Außenform des Mikrobehälters (3) mit Passung zur Innenform des Applikationskopfes (2) ausgebildet ist, oder
b) der Mikrobehälter von distal in den hohlen, hülsenförmigen Applikationskopf (2) eingebracht ist, wobei die Außenform des Mikrobehälters (3) zur Innenform des Applikationskopfes (2) durch eine Dichtung beabstandet ist.

10. Applikator nach einem der Ansprüche 1 bis 9, **dadurch gekennzeichnet, dass** die Innenform des hohlen, hülsenförmigen Applikationskopfs (2) in Passung zu der Außenform des Mikrobehälters (3) ausgebildet ist, so dass der Mikrobehälter (3) in den Applikationskopf (2) einschiebbar oder eindrehbar ist, bis die proximale Stirnseite (6) des Mikrobehälters (3) innen an den Sockel (8) des mindestens einen Stanzdorns oder an die proximale Innenwand des Applikationskopfes (2) anstößt, wobei a) der Winkel alpha (α) zwischen der Mittelachse (15) in der distalen Ebene (14) am distalen Bereich (2.2) des hohlen, hülsenförmigen Applikationskopfes (2a) gegenüber der Längsmittelachse (16) im proximalen Bereich (2.1) des Applikationskopfes mit alpha kleiner gleich 180° beträgt, insbesondere beträgt der Winkel alpha von 175° bis 110°, oder b) der Mikrobehälter (3) weist eine Soll-Knickstelle auf.

11. Applikator nach einem der Ansprüche 1 bis 10, **dadurch gekennzeichnet, dass**
i) die Folie oder die zerbrechliche Schicht aus einem der folgenden Materialien ausgewählt ist, umfassend Metalle, wie Aluminium, Legierungen, PET (Polyethylentherephthalat), PE (Polyethylen), PP (Polypropylen), COC (Cycloolefin-Copolymer), COP (Cycloolefin-Polymer), Acrylnitril-Butadien-Kautschuk, Ethylen-Vinylalkohol Polymer (EVOH, EVAL), halogenierte Polymere, Chloropren-Kautschuk, Perfluor-Kautschuk, PA (Polyamid), HDPE, LDPE, poröses expandiertes Polypropylen (PEPP) und/oder expandiertes Polypropylen (EPP), Glas, Hybridmaterial oder ein Verbundmaterial der vorgenannten Materialien, insbesondere ist die Folie oder die Schicht eine verschweißte oder kaschierte Aluminiumfolie, COC, COP oder PET-Folie, und/oder
ii) der Applikationskopf aus einem der folgenden Materialien ausgewählt ist, umfassend thermoplastische Elastomere (TPE), Silikon, Polyamid, Polyethylen, Polypropylen, Gummi oder ein Verbundmaterial der vorgenannten Materialien, insbesondere sind der Applikationskopf und die Filamente aus dem gleichen Material, und/oder
iii) das Material des Mikrobehälters (3) ist ausgewählt aus Polyethylen, Polypropylen, Ethylen-Vinylalkohol-Polymeren, Cycloolefin-Polymeren (COP) und/oder Cycloolefin-Copolymeren (COC), besonders bevorzugt Copolymere von Ethan mit Cycloolefinen, wie Norbonen, Norbornen-Derivat, Tetracyclododecen, oder Polymeren von Cycloocten, halogenierten Polymeren, HDPE, LDPE, poröses expandiertes Polypropylen (PEPP) und/oder expandiertes Polypropylen (EPP) und/oder metallischen Folien, und/oder
iv) das Material des mindestens einen Stanzdorns (4) und optional des Sockels (8) unabhängig voneinander aus einem der folgenden Materialien ausgewählt ist, aus Kunststoff mit einem Elastizitätsmodul von größer gleiche 1500 MPa nach ISO 527 oder größer gleich 1300 MPa nach ISO 178., Metall, Legierung, Glas, Keramik, Hybridwerkstoff.

12. Kit umfassend einen Mikro-Applikator nach einem der Ansprüche 1 bis 11, wobei in dem Mikrobehälter (3) eine Flüssigkeit enthalten ist, insbesondere eine mindestens ein organisches Lösemittel enthaltende Flüssigkeit, vorzugsweise eine flüssige dentale Zusammensetzung enthaltend mindestens ein flüchtiges organisches Lösemittel und optional mindestens ein Monomer sowie optional Wasser und/oder optional eine organische Säure.

13. Kit nach Anspruch 12, umfassend eine Umverpackung sowie eine Vielzahl an Mikro-Applikatoren.

14. Verwendung eines Mikro-Applikators nach einem der Ansprüche 1 bis 11 oder eines Kits nach Anspruch 12 oder 13 zur nicht-therapeutischen Applikation von flüssigen, dentalen Zusammensetzungen

## Claims

1. A dental micro-applicator (1) having a hollow sleeve-like application head (2) with a distal (2.2) and a proximal (2.1) region and with an application region (5.1) at the front face of the proximal region (2.1) of the application head (2),
wherein at least one punching spike (4), the tip (11) of which is oriented toward distal, is arranged in the hollow sleeve-like application head (2) in the proximal region (2.1), and an opening (7) having a cross section (d₁) is formed at the front face of the proximal region (2.1) for passage of a liquid or paste to be applied, a micro-container (3), the outer shape of which corresponds to the positive of the inner shape of the application head (2) being formed as the negative, being inserted, in particular slid in or turned in, from distal into the hollow sleeve-like application head (2), and the micro-container (3) having an internal space (10) to receive the liquid or paste to be applied, and the micro container (3) being closed at its distal side (9) and at its proximal front face (6), wherein the length (b) of the outer side wall (18) of the application head (2) to the length (c) of the outer side wall (12) of the micro-container (3) amounts to 1 to 1.2 to 1 to 25, wherein
the punching spike (4) with tip (11) has a proximal base (8), by which the punching spike (4) is fixed in the inner shape of the sleeve-like application head (2) in the proximal region of the application head (2) by means of a fit with transition fit or press fit of the outer shape of the base (8), wherein the punching spike (4) has a conduit (13), being continuous from distal to proximal, with cross section (d₄), wherein the cross section (d₁) of the opening of the application head (2) is greater than cross section (d₄).

2. The applicator according to claim 1, wherein the micro-container (3) is inserted, in particular slid in or turned in, inside the hollow application head (2) in transition fit or press fit by distance (I₀).

3. The applicator according to claim 2, wherein a) the micro-container (3) is slidable in inside the hollow application head (2) in transition fit or press fit by distance (I₁) additionally to distance (I₀), or b) a tubular micro-container is turnable in by a turning movement inside the hollow application head (2) in transition fit or press fit by distance (I₁) additionally to distance (I₀).

4. The applicator according to any of claims 1 to 3, wherein the micro-container (3) is closed by a film (6a) or a fragile layer (6b) at its proximal front face (6).

5. The applicator according to any of claims 1 to 4, wherein the inner shape of the hollow sleeve-like application head (2) is formed in fit to the outer shape of the micro-container (3) so that the micro-container (3) is slidable in or turnable in into the application head (2) until the proximal front face (6) of the micro container (3) abuts in the inside against the base (8) of the at least one punching spike (4) or against the proximal inside wall (19) of the application head (2).

6. The applicator according to claim 4 or 5, wherein the film (6a), or fragile layer (6b) at the proximal side of the micro-container (3), in particular of a tubular micro-container, is destroyed or cut off by at least one punching spike (4) when sliding in or turning in the micro-container into the application head (2).

7. The applicator according to any of claims 1 to 6, wherein a multitude of filaments (5), in particular having a minimal length (f) of 2 mm to 8 mm, in particular 3 mm to 7 mm, preferably 4 mm to 6 mm is formed in the application region (5.1) at the front face in the proximal region of the application head (2) to form a paintbrush (5.2).

8. The applicator according to claim 7, wherein the micro-container is a hollow section closed at the proximal and distal front face.

9. The applicator according to any of claims 1 to 8, wherein
a) the micro-container is inserted from distal into the hollow sleeve-like application head (2), the outer shape of the micro-container (3) being formed in fit to the outer shape of the application head (2), or
b) the micro-container is inserted from distal into the hollow sleeve-like application head (2), the outer shape of the micro-container (3) being spaced by a seal from the inner shape of the application head (2).

10. The applicator according to any of claims 1 to 9, wherein the inner shape of the hollow sleeve-like application head (2) is formed in fit to the outer shape of the micro-container (2) so that the micro-container (3) is slidable in or turnable in into the application head (2) until the proximal front face (6) of the micro-container (3) abuts in the inside against the base (8) of the at least one punching spike or against the proximal inside wall of the application head (2), wherein a) the angle alpha (α) between the central axis (15) in the distal plane (14) at the distal region (2.2) of the hollow sleeve-like application head (2) and the longitudinal central axis (16) in the proximal region (2.1) of the application head amounts to alpha less than or equal to 180°, in particular the angle alpha amounts from 175° to 110°, or b) the micro-container (3) has a predetermined kink point.

11. The applicator according to any of claims 1 to 10, wherein
i) the film or the fragile layer is selected from one of the following materials, comprising metals, such as aluminum, alloys, PET (polyethylene terephthalate), PE (polyethylene), PP (polypropylene), COC (cyclic olefin copolymer), COP (cyclic olefin polymer), acrylonitrile butadiene rubber, ethylene vinyl alcohol polymer (EVOH, EVAL), halogenated polymers, chloroprene rubber, perfluorinated rubber, PA (polyamide), HDPE, LDPE, porous expanded polypropylene (PEPP) and/or expanded polypropylene (EPP), glass, hybrid material or a composite material of the afore-mentioned materials, in particular the film or the layer is a welded or laminated aluminum film, COC, COP or PET film, and/or
ii) the application head is selected from one of the following materials, comprising thermoplastic elastomers (TPE), silicone, polyamide, polyethylene, polypropylene, rubber, or a composite material of the afore-mentioned materials, in particular the application head and the filaments are made of the same material, and/or
iii) the material of the micro-container (3) is selected from polyethylene, polypropylene, ethyl vinyl alcohol polymers, cyclic olefin polymers (COP) and/or cyclic olefin copolymers (COC), particularly preferably copolymers of ethane with cyclic olefins, such as norbornene, norbornene derivative, tetracyclododecene, or polymers of cyclooctene, halogenated polymers, HDPE, LDPE, porous expanded polypropylene (PEPP) and/or expanded polypropylene (EPP) and/or metallic films, and/or
iv) the material of the at least one punching spike (4) and optionally of the base (8), each independently, is selected from one of the following materials, from plastic having an elastic modulus of greater than or equal to 1500 MPa according to ISO 527 or greater than or equal to 1300 MPa according to ISO 178, metal, alloy, glass, ceramic, hybrid material.

12. A kit comprising a micro-applicator according to any of claims 1 to 11, a liquid being contained in the micro-applicator (3), in particular a liquid containing at least one organic solvent, preferably a liquid dental composition comprising at least one volatile organic solvent and optionally at least one monomer, and optionally water, and/or optionally an organic acid.

13. The kit according to claim 12, comprising an outer packaging as well as a multitude of micro-applicators.

14. Use of a micro-applicator according to any of claims 1 to 11, or of a kit according to claims 12 or 13 for non-therapeutic application of liquid dental compositions.

## Revendications

1. Micro-applicateur dentaire (1) avec une tête d'application (2) creuse en forme de manchon avec une zone distale (2.2) et une zone proximale (2.1) et avec une zone d'application (5.1) sur la face frontale de la zone proximale (2.1) de la tête d'application (2), au moins un poinçon (4), dont la pointe (11) est orientée vers le côté distal, étant disposé dans la tête d'application (2) creuse en forme de manchon dans la zone proximale (2.1), et la face frontale de la zone proximale (2.1), une ouverture (7) avec une section transversale (_{d1}) est formée pour le passage d'un liquide ou d'une pâte à appliquer, un microconteneur (3) étant introduit, en particulier inséré ou vissé, par la partie distale dans la tête d'application (2) creuse en forme de manchon, dont la forme extérieure correspond au positif de la forme intérieure, réalisée en négatif, de la tête d'application (2), et le microconteneur (3) présentant un espace intérieur (10) destiné à recevoir le liquide ou la pâte à appliquer, et, le microconteneur (3) étant fermé sur son côté distal (9) et sur son côté frontal proximal (6), la longueur (b) de la paroi latérale extérieure (18) de la tête d'application (2) par rapport à la longueur (c) de la paroi latérale extérieure (12) du microconteneur (3) présentant un rapport de 1 à 1,2 à 1 à 25,
le mandrin de poinçonnage (4) avec pointe (11) présentant un socle proximal (8) avec lequel le mandrin de poinçonnage (4) est fixé dans la zone proximale de la tête d'application (2) en forme de manchon par l'intermédiaire d'un ajustement avec ajustement transitoire ou ajustement serré de la forme extérieure du socle (8) dans la forme intérieure de la tête d'application (2) en forme de manchon et présentant un canal de section transversale (_{d4}) continu de distal en proximal, la section transversale (_{d1}) de l'ouverture de la tête d'application (2) étant plus grande que la section transversale (d₄).

2. Applicateur selon la revendication 1, **caractérisé en ce que** le microconteneur (3) est introduit, en particulier inséré ou vissé, selon la distance (_{I0}) à l'intérieur de la tête d'application creuse (2) en ajustement transitoire ou en ajustement serré.

3. Applicateur selon la revendication 2, **caractérisé en ce que** a) le microconteneur (3) peut être inséré selon la distance (_{I1}) en plus de la distance_{I0} à l'intérieur de la tête d'application creuse (2) en ajustement transitoire ou en ajustement serré ou b) un microconteneur tubulaire peut être tourné par un mouvement de rotation, selon la distance (_{I1}) en plus de la distance_{I0} à l'intérieur de la tête d'application creuse (2) en ajustement transitoire ou en ajustement serré.

4. Applicateur selon l'une des revendications 1 à 3, **caractérisé en ce que** le microconteneur (3) est fermé sur sa face frontale proximale (6) par un film (6a) ou une couche fragile (6b).

5. Applicateur selon l'une des revendications 1 à 4, **caractérisé en ce que** la forme intérieure de la tête d'application creuse en forme de manchon (2) est formée pour s'adapter à la forme extérieure du microconteneur (3), de sorte que le microconteneur (3) peut être inséré ou vissé dans la tête d'application (2) jusqu'à ce que la face frontale proximale (6) du microconteneur (3) vienne heurter intérieurement le socle (8) d'au moins un mandrin de poinçonnage (4) ou la paroi intérieure proximale (19) de la tête d'application (2).

6. Applicateur selon la revendication 4 ou 5, **caractérisé en ce que** le film (6a), ou la couche fragile (6b) sur la face proximale du microconteneur (3), en particulier d'un microconteneur tubulaire, est détruit ou séparé par au moins un poinçon (4) lors de l'insertion ou du vissage du microconteneur dans la tête d'application (2).

7. Applicateur selon l'une des revendications 1 à 6, **caractérisé en ce que** dans la zone d'application (5.1) sur la face frontale, dans la zone proximale de la tête d'application (2), une pluralité de filaments (5) sont formés pour former un pinceau (5.2), en particulier avec une longueur minimale (f) de 2 mm à 8 mm, en particulier de 3 mm à 7 mm, de préférence de 4 mm à 6 mm .

8. Applicateur selon la revendication 7, **caractérisé en ce que** le microconteneur est un profilé creux fermé sur les faces frontales proximale et distale.

9. Applicateur selon l'une quelconque des revendications 1 à 8, **caractérisé en ce que** a) le microconteneur est inséré de manière distale dans la tête d'application creuse en forme de manchon (2), la forme extérieure du microconteneur (3) étant formée avec un ajustement à la forme intérieure de la tête d'application (2), ou
b) le microconteneur est inséré par la partie distale dans la tête d'application creuse en forme de manchon (2), la forme extérieure du microconteneur (3) étant espacée de la forme intérieure de la tête d'application (2) par un joint d'étanchéité.

10. Applicateur selon l'une quelconque des revendications 1 à 9, **caractérisé en ce que** la forme intérieure de la tête d'application creuse en forme de manchon (2) est formée pour s'adapter à la forme extérieure du microconteneur (3), de sorte que le microconteneur (3) peut être inséré ou tourné dans la tête d'application (2), jusqu'à ce que la face frontale proximale (6) du microconteneur (3) vienne heurter intérieurement le socle (8) d'au moins un mandrin de poinçonnage ou la paroi intérieure proximale de la tête d'application (2), dans lequel a) l'angle alpha (α) entre l'axe central (15) dans le plan distal (14) au niveau de la partie distale (2.2) de la tête d'application creuse en forme de manchon (2a) par rapport à l'axe médian longitudinal (16) dans la zone proximale (2.1) de la tête d'application avec alpha est inférieur ou égal à 180°, en particulier l'angle alpha est compris entre 175° et 110°, ou b) le microconteneur (3) présente un point de pliage théorique.

11. Applicateur selon l'une quelconque des revendications 1 à 10, **caractérisé en ce que** i) le film ou la couche fragile est choisi parmi l'un des matériaux suivants, comprenant des métaux, tels que l'aluminium, des alliages, le PET (polyéthylène téréphtalate), PE (polyéthylène), PP (polypropylène), COC (copolymère de cyclooléfine), COP (polymère de cyclooléfine), caoutchouc d'acrylonitrile-butadiène, polymère d'éthylène-alcool vinylique (EVOH, EVAL), polymères halogénés, caoutchouc de chloroprène, caoutchouc perfluoré, PA (polyamide), HDPE, LDPE, polypropylène expansé poreux (PEPP) et/ou polypropylène expansé (EPP), verre, matériau hybride ou matériau composite des matériaux susmentionnés, en particulier le film ou la couche est une feuille d'aluminium, COC, COP ou PET soudée ou contrecollée, et/ou ii) la tête d'application est choisie parmi l'un des matériaux suivants, comprenant des élastomères thermoplastiques (TPE), du silicone, du polyamide, du polyéthylène, du polypropylène, du caoutchouc ou un matériau composite des matériaux précités, en particulier la tête d'application et les filaments sont faits du même matériau, et/ou iii) le matériau du microconteneur (3) est choisi parmi le polyéthylène, le polypropylène, les polymères d'éthylène et d'alcool vinylique, les polymères de cyclooléfine (COP) et/ou les copolymères de cyclooléfine (COC), de préférence les copolymères d'éthane avec des cyclooléfines, comme le norbornène, le dérivé de norbornène, le tétracyclododécène, ou des polymères de cyclooctène, des polymères halogénés, du HDPE, du LDPE, du polypropylène expansé poreux (PEPP) et/ou du polypropylène expansé (EPP) et/ou des feuilles métalliques, et/ou iv) le matériau d'au moins un mandrin de poinçonnage (4) et éventuellement de la base (8) est choisi indépendamment parmi l'un des matériaux suivants, en plastique avec un module d'élasticité supérieur ou égal à 1500 MPa selon ISO 527 ou supérieur ou égal à 1300 MPa selon ISO 178.le matériau de base est un métal, un alliage, un verre, une céramique ou un matériau hybride.

12. Kit comprenant un micro-applicateur selon l'une des revendications 1 à 11, dans lequel un liquide est contenu dans le microconteneur (3), en particulier un liquide contenant au moins un solvant organique, de préférence une composition dentaire liquide contenant au moins un solvant organique volatil et éventuellement au moins un monomère, et éventuellement de l'eau et/ou éventuellement un acide organique.

13. Kit selon la revendication 12, comprenant un suremballage ainsi qu'une pluralité de micro-applicateurs.

14. Utilisation d'un micro-applicateur selon l'une des revendications 1 à 11 ou d'un kit selon la revendication 12 ou 13 pour l'application non thérapeutique de compositions dentaires liquides.
